# EUROPÄISCHE PATENTSCHRIFT

(11) **EP 1 631 373 B1**
(45) Veröffentlichungstag und Bekanntmachung des Hinweises auf die Patenterteilung: **24.12.2008**
(21) Anmeldenummer: 04735882.5
(22) Anmeldetag: 03.06.2004
(51) Int. Cl.: B01J 2/16, A61K 31/7048

(54) **MIKROPELLETS, VERFAHREN ZUR IHRER HERSTELLUNG SOWIE DEREN VERWENDUNG**
MICROPELLETS, METHOD FOR THE PRODUCTION THEREOF, AND USE THEREOF
MICROPELLETS, LEURS PROCEDES DE PRODUCTION ET LEUR UTILISATION

(30) Priorität: 07.06.2003 DE 10325989
(43) Veröffentlichungstag der Anmeldung: 08.03.2006
(73) Patentinhaber: GLATT GMBH, 79589 Binzen (DE)
(72) Erfinder: PRASCH, Armin, 79100 Freiburg (DE); LUY, Bernhard, 79295 Sulzburg (DE); PÖLLINGER, Norbert, 79379 Müllheim (DE); STRUSCHKA, Manfred, 79424 Auggen (DE); SCHWARZ, Franz, X., A-6300 Wörgl (AT)
(74) Vertreter: Maucher, Wolfgang
(86) Internationale Anmeldenummer: PCT/EP2004/005993
(87) Internationale Veröffentlichungsnummer: WO 2004/108266

(56) Entgegenhaltungen:
- EP-A- 1 027 887
- WO-A-02/089773
- WO-A-02/092106
- US-A- 6 013 280

## Beschreibung

### Zusammenfassung der Erfindung

Die Erfindung betrifft ein Verfahren zur Herstellung von Mikropellets, umfassend schwer wasserlösliche Wirkstoffe in Form einer festen Dispersion, damit erhältliche Mikropellets, Verfahren zur Herstellung von Dispersionen von schwerlöslichen Wirkstoffen, wobei die Dispersionen insbesondere für die Herstellung der Mikropellets verwendet werden, pharmazeutische Formulierungen, welche die genannten Mikropellets beinhalten, sowie die Verwendung der Mikropellets zur Herstellung beschichteter Mikropellets und/oder derartiger Formulierungen, jeweils wie in den Ansprüchen beschrieben. Die Mikropellets (unbeschichtet auch als Mikropelletkerne zu bezeichnen) beinhalten insbesondere pharmazeutische Wirkstoffe.

### Hintergrund der Erfindung

Pharmazeutische, enteral anwendbare Darreichungsformen sollen für die jeweilige Anwendung in geeigneter Weise formuliert sein, um eine Freisetzung der pharmazeutischen Wirkstoffe zum richtigen Zeitpunkt und ohne störende Nebeneffekte zu bewirken. So sollen beispielsweise oral verabreichbare Wirkstoffe möglichst so freigesetzt werden, dass kein unangenehmer (z.B. bitterer) Geschmack im Mund entsteht, der insbesondere bei Kindern zu Abwehrreaktionen führen und so die "Compliance" beeinträchtigen würde. Andererseits müssen die Wirkstoffe jedoch im Magen oder Darm möglichst vollständig und in rasch resorbierbarer Form freigesetzt werden, wenn eine systemische Behandlung erfolgen soll.

So besteht ein Bedürfnis, Granulate, wie Mikrosphärulen, die pharmazeutische Wirkstoffe enthalten, herzustellen, die eine derartige Beschichtung (Mikroverkapselung) erlauben.

Bei klassischen Verfahren zur Mikroverkapselung von schwerlöslichen Wirkstoffen ist dies nur unter großen Schwierigkeiten zu erreichen. Dies gilt insbesondere bei der Herstellung von Granulaten aus mehreren Komponenten (Wirkstoffe, Hilfsstoffe), insbesondere im Falle schwerlöslicher Wirkstoffe.

Bei W/O/W'-Emulsionsverfahren (W steht für eine wässrige, O für eine lipophile Phase) wird eine wässrige Wirkstofflösung mit einer Lösung eines Polymers in einem mit Wasser nicht mischbaren organischen Lösungsmittel emulgiert. Diese W/O-Emulsion wird dann in einem großen Volumen einer beispielsweise polyvinylalkoholhaltigen W'-Phase dispergiert. Das apolare Polymerlösungsmittel verteilt sich in die Wasserphase und das Polymer wird als Koazervat ausgefällt. Bei der Phasenseparationstechnik dagegen wird zu einer Dispersion oder Emulsion des Wirkstoffs ein Phasentrennmittel (z.B. Silikonöl) gegeben, welches eine Polymer-Koazervation des Lösungsmittels auf dem Wirkstoff bewirkt. Nach der Bildung der Mikropartikel mittels W/O/W'-Emulsion oder Phasenseparation werden sie auf übliche Weise gehärtet, filtriert und gewaschen. Diese Verfahren belasten die Formulierungen mechanisch und chemisch. Beim Extrusionsverfahren wird eine Pulvermischung aus Wirkstoff, Polymer und weiteren Hilfsstoffen erhitzt durch eine Düse gepresst. Dabei wird ein Zylinderkörper gebildet, der weiter verarbeitet werden kann. Wegen der erforderlichen hohen Temperaturen können die Wirkstoffe mit dem Polymer interagieren, und es kann zu Abbauprozessen kommen. Hier, wie auch im Falle der Feuchtgranulierungsmethoden, gelingt es nur sehr schwer, Inhomogenitäten im wirkstoffhaltigen Kern der gebildeten Partikel zu vermeiden. Der Grund hierfür sind in erster Linie Entmischungsphänomene der beteiligten Komponenten, doch spielen auch andere Phänomene eine negative Rolle, bis hin zur Bildung von Aggregationen der Komponenten, die eine Wirkstoff-Freisetzung behindern.

Eine Mikronisierung ist insbesondere für schwer (insbesondere wasser-) lösliche (insbesondere pharmazeutische) Wirkstoffe von Vorteil, da die spezifische Partikeloberfläche umso größer wird, je kleiner die Einzelabmessung eines Partikels ist. Da sämtliche Austauschvorgänge (Stoffaustausch und/oder Wärmeübergang) bei Partikeln direkt proportional zur Partikeloberfläche stattfinden, wird dadurch auch das Löslichkeitsverhalten und damit letztlich auch die Bioverfügbarkeit beeinflusst. Liegen insbesondere nur schwer wasserlösliche Wirkstoffe in mikronisierter Form vor, so kann die Löslichkeit durch eine möglichst große Stoffaustauschfläche (= Partikeloberfläche) deutlich verbessert werden, was die Vorteile mikronisierter Materialien belegt.

Mit klassischen Verfahren zur Herstellung von festen pharmazeutischen Formulierungen lassen sich insbesondere kleinere Partikel von Wirkstoffen unter anderem aufgrund ihrer geringen Schüttdichte, elektrostatischer Aufladung etc. nur unter großen Schwierigkeiten verarbeiten. So lässt sich ein mikronisierter Stoff normalerweise nicht oder nur sehr eingeschränkt weiterverarbeiten, denn aufgrund der geringen Korngröße entsteht eine starke Neigung zur Staubbildung, die Rieselfähigkeit fehlt und die Mischung mit anderen Feststoffen, die als Formulierungsbestandteile eines pharmazeutischen Produktes erforderlich sind, ist nur sehr schwer möglich. Die Schüttdichte von mikronisiertem Material liegt typischerweise bei < 0,2 kg/l, oft sogar nur im Bereich von 0,1 kg/l. Eine Vermischung eines nur schwer wasserlöslichen mikronisierten Wirkstoffes mit einem Lösungsmittel (z.B. Wasser) mittels eines konventionellen Rührers (z.B. Flügelradrührer) führt weiterhin in der Regel zu starker Schaumbildung und aufgrund des großen Dichteunterschiedes auch zur Separierung von Flüssigkeit und Feststoff. Eine möglichst homogene Mischung oder Dispersion kann so nicht hergestellt werden. Daher entstehen Probleme beispielsweise bei der Produkthandhabung (Dosieren, Abfüllen und dergleichen), beim Mischen mit weiteren Hilfsstoffen oder beim Beschichten, um die Geschmacksabdichtung ("Taste Masking") und eine pH-abhängige, kontrollierte Freisetzung zu gewährleisten.

Auch ist es bei diesen klassischen Methoden schwierig, Granulatkerne von gleichmäßiger Größe und von möglichst ideal runder Form zu gewinnen, die es beispielsweise ermöglichen, eine optimale Beschichtung mit anderen Komponenten, wie beispielsweise geschmacksabdichtenden Beschichtungen ("Taste-masking Coatings") und/oder Schutzbeschichtungen (beispielsweise magensaftresistenten Beschichtungen) ("Protection Coating") vorzunehmen. Aus der EP 0 163 836 sind Verfahren zur Herstellung von vor allem für Agrochemikalien eingesetzten Granulaten mit engen Korngrößenverteilungen bekannt, die nach einem Wirbelschichtverfahren hergestellt werden. Diese sind nach den Beispielen entweder unter Verwendung der reinen Wirkstoffe hergestellt oder unter Verwendung von Lösungen, Schmelzen oder Suspensionen, die inerte Füllmaterialien, Dispergier- und/oder Bindemittel sowie Zusatzstoffe optional enthalten können. EP 1 027 887 und WO 02/089773 beschreiben Pellets mit schwerlöslichen Antibiotika, die relativ komplex sind und im Falle der letzteren nicht mittels Wirbelschicht hergestellt werden.

Aufgabe der vorliegenden Erfindung ist es daher, Mikropellets herzustellen, die eine optimale Form, Größe und Homogenität der Matrix aufweisen, um die Herstellung beschichteter Mikropellets zu ermöglichen, die schwer wasserlösliche Wirkstoffe umfassen und die genannten Probleme und Nachteile vermeiden helfen und weitere Vorteile aufweisen.

### Allgemeine Beschreibung der Erfindung

Die Aufgabe wird gelöst durch eine Methode oder ein Verfahren gemäß Anspruch 1 zur Herstellung von einen oder mehrere schwerlösliche Wirkstoffe enthaltenden Mikropellets, bei dem aus Dispersionen mikronisierter Teilchen in Gegenwart eines funktionellen Hilfsmittels zur Formung einer festen Dispersion derartiger Teilchen, wobei diese funktionellen Hilfsmittel und die übrigen Komponenten zur Bildung der Mikropellets in gelöster oder ebenfalls dispergierter Form vorliegen, durch Sprühgranulation im Wirbelschichtverfahren Mikropellets hergestellt werden.

Dies hat unter anderem folgende Vorteile gegenüber dem eingangs genannten Stand der Technik: Zum einen ist kein vorzulegendes Inertmaterial als Granulationskeim erforderlich. Darüber hinaus haben die resultierenden Mikropellets eine Fülle von weiteren Vorteilen, deren Kombination überrascht, beispielsweise einen sehr homogenen Matrixaufbau, hohe Abriebfestigkeit und geringe Staubbildung bei ihrer Herstellung (so dass keine nicht beispielsweise bezüglich des Geschmacks kaum abdeckbare Staubpartikeln entstehen). Darüber hinaus bietet das erfindungsgemäße Verfahren den Vorteil, dass ein hoher Wirkstoffgehalt bzw. Gehalt an erforderlichen funktionellen Hilfsmitteln zur Formung einer festen Dispersion möglich ist. Weiter resultieren praktisch ideal kugelförmige, sphärische Mikropellets, die besonders geeignet sind für ein späteres Beschichten ("Coating"). Es entstehen homogene Mikropellets mit hoher Dichte und entsprechend geringer Porosität. Die Mikropellets sind sehr abriebsstabil - abgeriebene Partikel werden gemäß dem Verfahrensprinzip sofort wieder im Mikropelletkern fixiert. Unter anderem die hohe Abriebfestigkeit und die resultierende geringe Staubbildung ermöglichen eine sehr enge Größenverteilung der Teilchen ohne zusätzliche Siebung der Mikropellets nach dem Pelletieren (z.B. zwischen 200 und 400 µM), was wiederum eine besonders große Eignung für späteres Beschichten bewirkt. Beispielsweise ist es ohne weiteres möglich, zu erreichen, dass nur maximal 25 Gew.-% der Mikropellets einen Durchmesser aufweisen, der um mehr als 25 % (±) vom mittleren Durchmesser aller Mikropellets abweicht. Das Endprodukt ist, da nicht extern gesichtet, staubfrei, was wiederum eine ideale Voraussetzung für späteres Beschichten darstellt. Die Gesamtausbeute ist sehr hoch, beispielsweise gemessen an der Teilchengrößenverteilung 85 oder mehr %, beispielsweise größer 95 %.

Hauptvorteil ist, dass die Mikropellets eine maximale Homogenität und eine ideale Eignung zur Auftragung (auch mehrerer) Beschichtungen aufweisen. Die feste Dispersion des oder der Wirkstoffe als besonders wichtiges Merkmal der nach dem erfindungsgemäßen Verfahren erhältlichen Mikropellets bewirkt eine bedeutend erhöhte Bioverfügbarkeit.

Somit sind völlig neue Produktmerkmale das Ergebnis dieses überraschend einfachen Verfahrens. Auch die Verarbeitung sonst, wie eingangs für kleinere Wirkstoffpartikel erwähnt, ganz besonders schwierig zu behandelnder mikronisierter Wirkstoffpartikel wird ohne weiteres möglich.

Die mittels des genannten Verfahrens erhältlichen Mikropellets selbst bilden einen weiteren Gegenstand der Erfindung - sie beinhalten einen oder mehrere schwerlösliche Wirkstoffe in mikronisierter Form sowie ein oder mehrere funktionelle Hilfsmittel zur Formung einer festen Dispersion derartiger Wirkstoffe.

Die Mikropellets eignen sich insbesondere für die Darstellung von entereal, insbesondere oral, verabreichbaren pharmazeutischen Formulierungen, einerseits durch Verarbeiten zu Tabletten, beispielsweise Dragees, oder Trockenkapseln, andererseits nach Beschichtung in Form wässriger Suspensionen oder deren Vorstufen in Trockenform, die durch Zugabe von wässrigen Lösungen oder Wasser suspendiert werden können, so dass derartige Formulierungen ebenfalls Gegenstand der Erfindung sind.

### Beschreibung der Zeichnung

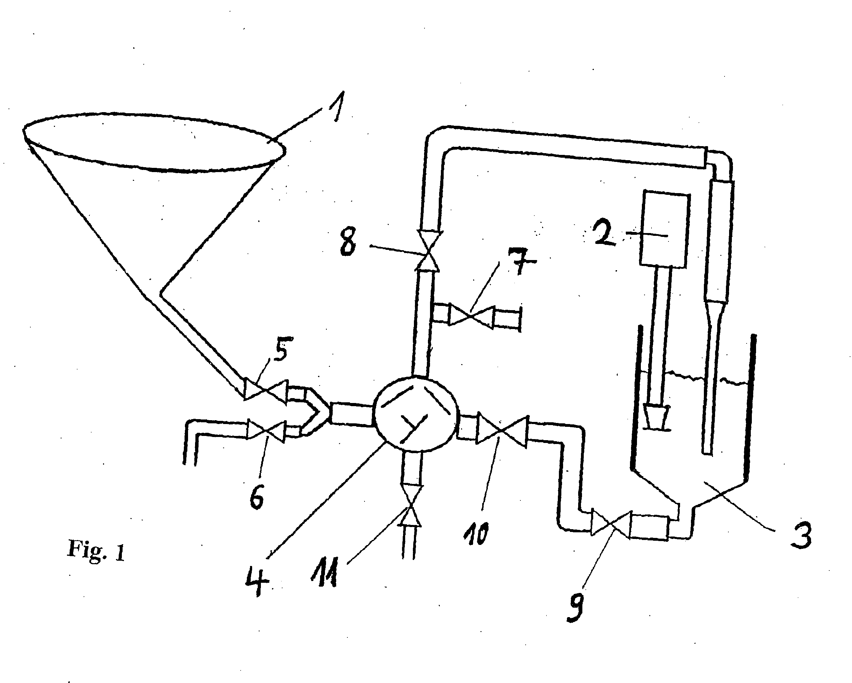

Fig. 1 zeigt schematisch ein Beispiel für verwendete Geräte zur Herstellung einer Dispersion eines schwer wasserlöslichen mikronisierten Wirkstoffs.

**1** Pulverzufuhr, hier als Pulvertrichter ausgebildet; **2** Leitstrahlmischer; **3** Ansatzbehälter; **4** Pulverbenetzunsgmaschine; **5, 6, 7, 8, 9, 10** und **11** = Ventile: **5** Ventil, beispielsweise Kugelventil, zum Einziehen des Pulvers in die Anlage; **6** = optionales Steuerventil, beispielsweise als Kugelventil ausgeführt, zur Zuführung einer weiteren Flüssigkeit oder eines weiteren festen Stoffs während des Dispergiervorgangs, vorzugsweise schnell öffen-. und schließbar, auch Zwischenzustände möglich; **7 =** optionales Ventil, insbesondere Klappenventil, zum Abpumpen von Reinigungsmedium nach Reinigung der Anlage, vorzugsweise angeschlossen an ein Schlauch- oder Rohrsystem zur Entsorgung der Reinigungsflüssigkeit, mögliche Zustände vorzugsweise offen oder geschlossen; **8** optionales bei der Reinigung zu verschließendes (beispielsweise Klappen-)ventil, mögliche Zustände vorzugsweise offen oder geschlossen; **9** und **10** jeweils optionale Serviceventile, bei Bedarf zu schließen, um zu verhindern, dass bereits im Behälter 3 befindliches Produkt im Falle von Servicearbeiten an der Pulverbenetzungsmaschine zuvor abgelassen werden muss, vorzugsweise als Klappenventil ausgeführt und vorzugsweise mit den Stellungen offen oder geschlossen; **11** optionales Ventil, beispielsweise Klappenventil, zur Restentleerung des Pumpenkopfes und des Systems nach der Umfüllphase, Stellungen vorzugsweise entweder offen oder geschlossen.

### Detaillierte Beschreibung der Erfindung

Detaillierte und bevorzugte Ausführungen der Erfindung ergeben sich speziell aus den Beispielen und allgemein aus den Ansprüchen, letztere werden hier durch Bezugnahme aufgenommen, sowie aus den nachfolgenden Erläuterungen:

In den erfindungsgemäßen Mikropellets liegen ein oder mehrere pharmakologisch wirksame Wirkstoffe in mikronisierter Form in einem Anteil von 10 bis 99 Gew.-%, vorzugsweise von 20 bis 90 Gew.-%; funktionelle Hilfsmittel zur Formung der festen Dispersion in einem Anteil von 1 bis 90 Gew.-%, vorzugsweise von 1 bis 50 Gew.-%, und gewünschtenfalls ein Bindemittel in einem Anteil von 0 bis 20 Gew.-%, beispielsweise von 5 bis 15 Gew.-%, vor, wobei die Summe dieser Komponenten 100 Gew.-% ergibt.

Als schwer oder nicht wasserlöslicher Wirkstoff eignen sich beispielsweise, ohne dass diese Aufzählung erschöpfend sein soll, ein oder mehrere der in der Roten Liste 2003 für Arzneimittel; Editio Cantor Verlag, Aulendorf 2003 (hier durch Bezugnahme aufgenommen) genannten Wirkstoffe, und/oder insbesondere einer oder mehrere der folgenden im verwendeten Lösungsmittel (insbesondere einer wässrigen Lösung) schwerlöslichen Wirkstoffe:
- Antibiotika, insbesondere Macrolidantibiotika, wie Clarithromycin, Erythromycin, Azithromycin, Roxithromycin, Spiramycin oder Josamycin, ferner Ketolide, wie Telithromycin, ferner
- schwer wasserlösliche antivirale Therapeutika, z.B. antiretrovirale Proteasehemmer, wie Indinavir, Saquinavir, Ritonavir oder Nelfinavir;
- Analgetika, wie Paracetamol;
- Herz-Kreislaufmittel, z.B. Ca-Antagonisten, wie Nifedipin;
- Antiphlogistika, wie Glucocorticoide, z.B. Cortison, Prednisolon oder Prednisolonacetat,
- Krebstherapeutika, wie Mitosehemmstoffe, beispielsweise Hemmstoffe der Mikrotubuli-Desaggregation, wie Taxane, z.B. Paclitaxel oder Docetaxel,
oder dergleichen.

Funktionelle Hilfsmittel zur Formung der festen Dispersion der genannten Wirkstoffe sind vorzugsweise Lösungsvermittler, insbesondere Polyoxypropylenpolyoxyethylenkondensate oder -blockpolymer-isate, wie Poloxamer, z.B. Pluronic® (Marke der BASF), Fettsäurepolyglykolether, wie Lösungsvermittler K 2® (General Mills, USA), Alkylphenol-polyethylenglykolether, wie Lösungsvermittler S-12 (Givaudan), Triglyceride, wie "Labrafil M 2375®" (Polyoxyethylenglycerintrioleat der Firma Gattefossé, Paris), "Miglyol 812®" (Triglycerid gesättigter Fettsäuren der Kettenlänge C₈ bis C₁₂ der Firma Hüls AG, Deutschland), oder Tenside, wie anionische Tenside, die i.d.R. langkettige Fettsäuren als hydrophoben Anteil haben, wie insbesondere Sulfate langkettiger (vor allem C₈-C₁₈-) Alkohole, z.B. Alkalimetall-C₈-C₁₈-Alkanoylsulfate, wie insbesondere Natriumdodecylsulfat oder Natriumtridecylsulfat; die Sulfate oder Sulfonate von Monoglyceriden von Fettsäuren, wie Alkalimetall- (insbesondere Natrium-)glycerylsulfat oder Natrium-Kokosnußöl-monoglyceridsulfonat; die Sulfonate von Succinsäureestern, wie Natrium-dioctylsulfosuccinat; die Alkylsulfoacetate wie Natriumlauroylsulfoacetat oder Natrium-Kokosnußöl-sulfoacetat; Salze von Sulfoessigsäure, modifiziert durch Aminoethyl-langkettige-Fettsäureester, wie Natriumsulfocolaurat; die Amide höherer Fettsäuren mit kurzkettigen aliphatischen Aminosäuren, wie Natriumlauroylsarcosinat oder Natriummethyllauroyltaurid; und Seifen wie die Natrium-, Kalium- oder Triethanolaminsalze von Fettsäuren, beispielsweise von Laurinsäure, Myristinsäure, Palmitinsäure, Stearinsäure oder Mischungen davon, oder Kokosnußölfettsäuren oder Talgfettsäuren; kationische Tenside, welche neben hydrophoben aliphatischen, aromatischen oder Alkyl-Resten eine positiv geladene hydrophile Gruppe (meist quaternäres Ammonium) aufweisen, z.B. die (zusätzlich antibakteriell wirksamen) Tenside Benzyl-dimethylstearylammoniumchlorid oder Cetylpyridiniumchlorid; amphotere Detergentien, wie mono- oder dicarboxylierte Imidazoline von Fettsäuren, wie Natriumlauryldicarboxyimidazolin oder Natrium-Kokosnußöl-dicarboxyimidazolin, oder Triazaeicosancarbonsäure; oder nichtionische Tenside, wie ethoxylierte Zuckerester höherer Fettsäuren, beispielsweise Polyoxyethylensorbitan-monolaurat, -palmitat, -stearat, -tristearat, -monooleat oder -trioleat, oder alternativ oder ergänzend andere Lösungsvermittler, wie z.B. weitere in Fiedler, "Lexikon der Hilfssstoffe für die Pharmazie, Kosmetik und angrenzende Gebiete", Editio Cantor Verlag, 5. Auflage, Aulendorf 2002, S. 1060-1061, genannte, welche hier durch Bezugnahme aufgenommen werden; oder (weniger bevorzugt) Gemische von zwei oder mehr davon, soweit sie mischbar sind (beispielsweise sind anionische Tenside nicht gut mischbar mit kationischen Tensiden).

Bindemittel sind insbesondere in Granulaten übliche natürliche oder synthetische Bindemittel ("Kleber"), z.B. Hydroxyalkylcellulosen, wie Hydroxymethylcellulose und Hydroxyethylcellulose; Methylcellulose; Pflanzengummen wie Tragant, Gummi arabicum, Carayagummi, Guargummi, Xanthangummi und Irish Moss; Polyvinylpyrrolidon, Polyvinylalkohol, Polyvinylacetat, Gelatine, Stärke, Carboxymethylstärke; speziell hydrierte Kolophonium-Ester; Polyurethane; synthetische Polyelektrolyte, wie Alkalisalze der Polyacrylsäure; Polyethylenglykole mit einem Molekulargewicht um 900 oder darüber, z.B. Carbowax^{®} 900, 1000, 1450, 3350, 4600 oder 8000) anorganische Verdickungsmittel, beispielsweise anorganisches amorphes Siliciumdioxid, wie Hydrogele (z.B. Sylodent^{®} 15 oder Sylodent^{®} 2 von W.R. Grace and Co.), pyrogenes, sublimiertes oder Schwebeteilchen-Siliciumdioxid (wie Aerosil^{®} 200 von Degussa oder Cabosil^{®} von Cabot), kolloidales Magnesiumaluminiumsilikat, disperses Siliciumoxid, kolloidales Siliciumoxid oder Gemische von zwei oder mehreren dieser Bindemittel, vorzugsweise nur eines dieser Bindemittel.

Mikronisiert bedeutet, dass der oder die Wirkstoff(e) in stark zerkleinerter Form eingesetzt wird/werden. Bevorzugt sind Teilchengrößen unterhalb von 30 µm, beispielsweise zwischen 0,1 und 30 µm. Die Mikronisierung des Wirkstoffs erfolgt beispielsweise durch Vermahlen mit geeigneten Mühlen. Besonders geeignet sind beispielsweise Luftstrahlmühlen (bereits vorverkleinertes pulverförmiges Mahlgut wird gemeinsam mit einem Gas (Luft, evtl. Inertgas gegen Staubexploxionen, wie Stickstoff oder Argon) unter erhöhtem Druck (beispielsweise 10 bar) tangential in die kreisrunde Mahlkammer eingeblasen; durch das expandierende Gas werden die Pulverteilchen stark beschleunigt (beispielsweise 300 m/s) und rotieren in der Mahlkammer durch die Zentrifugalkraft in Abhängigkeit von ihrer Masse mehr öder weniger am Rande der Mahlkammer; durch gegenseitige Reibung und Prallvorgänge (Strahlmählung) werden die Teilchen weiter zerkleinert, bis sie so fein (mikronisiert) sind, (10 bis 0,1 µm, Mikropulver), dass sie mit dem Gas im Zentrum aus dem Mahlraum ausgetragen und in Filtersäcken abgeschieden werden; der durch die Gasexpansion auftretende Kühleffekt ermöglicht auch die Mikronisierung thermolabiler Verbindungen) oder Kolloidmühlen (hier bewegt sich ein konischer Rotor mit großer Geschwindigkeit in einem regelbaren Abstand (Bruchteile eines Millimeters) zum Mühlengehäuse. Das Mahlgut wird in Wasser suspendiert zugeführt, passiert den feinen Spalt und wird durch die Scherkräfte zwischen mit Wasser benetztem Rotor und Stator (Gehäusewand) zerkleinert. Die herstellbaren Teilchengrößen liegen in günstigen Fällen unter 0,1 µm.

Das Verfahren zur Herstellung eines Mikropellets ist insbesondere dadurch gekennzeichnet, dass eine flüssige, vorzugsweise wässrige Dispersion eines oder mehrerer schwerlöslicher mikronisierter Wirkstoffe die ferner ein oder mehrere funktionelle Hilfsmittel zur Formung einer festen Dispersion (vorzugsweise in gelöster Form) und gewünschtenfalls ein oder mehrere Bindemittel (Vorzugsweise in gelöster Form) beinhaltet, von unten in eine zu Beginn des Prozesses leere Wirbelschichtanlage eingesprüht wird; durch die Sprühgranulation der Dispersion selbsttätig Startkeime für die Pelletisierung ohne Vorlage von inertem Material gebildet werden;
und die hergestellten Pellets während des Prozesses über eine Klassiereinrichtung, insbesondere einen Windsichter, in erster Linie einen Zick-Zack-Sichter nach EP 0 332 031 B1 (dieses Patent wird hier diesbezüglich durch Bezugnahme aufgenommen) gesichtet und bei Erreichen einer vordefinierten Pelletgröße aus dem Sichter entnommen werden.

Die flüssige, vorzugsweise wässrige Dispersion wird vorzugsweise wie folgt hergestellt, wobei dieses Herstellungsverfähren für die Dispersion eine besonders bevorzugte eigene Erfindung darstellt:

In einem ersten separaten Schritt wird eine homogene Suspension des mikronisierten Wirkstoffs in Wasser hergestellt, indem der mikronisierte schwerlösliche, insbesondere nicht wasserlösliche Wirkstoff, mehrere entsprechende Wirkstoffe oder ein entsprechendes Wirkstoffgemisch mittels einer Pulverbenetzungs- oder Dispergiermaschine, beispielsweise Ystral CONTI TDS-2 (Ystral GmbH Maschinenbau und Processtechnik, Ballrechten-Dottingen, Deutschland) und einem Mischer zum Homogenisieren bzw. Entlüften der Dispersion, z.B. einem Leitstrahlmischer der Firma Ystral oder einem Ultra-Turrax der Firma Jahnke & Kunkel (Staufen, Deutschland), in Wasser suspendiert wird. Hierbei ist darauf zu achten, dass die Mischung gleichzeitig entlüftet und homogenisiert wird und die mikronisierten Feststoffpartikel nicht agglomerieren, sondern in der mikronisierten Größe der Ausgangspartikel gleichmäßig verteilt in der Dispersion vorliegen. Dies gelingt insbesondere, indem für die Entlüftung in einer relativ kleinen Mischkammer (beispielsweise mit einem Volumen von 10 bis 500 ml, z.B. ungefähr 200 ml) ein geringer Anteil Wirkstoff in viel Flüssigkeit aufgenommen wird. Insgesamt ist im Gesamtansatz einerseits der mechanische Energieeintrag für gleichmäßige Homogenisierung wichtig, der umso besser wird, je höher die Feststoffkonzentration ist. Andererseits führt ein zu hoher Feststoffgehalt zu geringerer Prozesssicherheit. Nach Zufuhr der gesamten Wirkstoffmenge ist im Gesamtansatz beispielsweise ein Gewichtsverhältnis im Bereich von 1:1 (1 Anteil Wirkstoff auf 1 Anteil Flüssigkeit) bis 1:3 zu bevorzugen, wobei in einer besonderen Ausführungsform der Erfindung dieses Verhältnis im Bereich von 1:1,5 bis 1:2,5 liegt, beispielsweise bei 1:2 bis 1:2,2. Nach dem Zuführen des Wirkstoffs erfolgt die Überführung in einen größeren Behälter, und es folgt vorzugsweise weitere Entlüftung mit einem Leitstrahlmischer, wobei darauf geachtet werden muss, dass keine zusätzliche Luft eingeschlagen wird.

In einem separaten weiteren Schritt wird eine Lösung der löslichen (insbesondere wasserlöslichen) funktionellen Hilfsmittel und übrigen Komponenten zur Bildung eines Mikropellets, wie jeweils insbesondere vor- und nachstehend näher definiert, in einem (insbesondere wässrigen) Lösungsmittel hergestellt, bis die Lösung klar wird. Dies kann auf konventionellem Wege, beispielsweise mittels eine Flügelradrührers oder eines Rührers mit Dissolverscheibe geschehen. Sobald eine klare Lösung vorliegt, sind die funktionellen Hilfsmittel und übrigen Komponenten in der Lösung homogen verteilt.

Die Dispersion aus dem ersten Schritt und die homogene Lösung aus dem weiteren Schritt (ggf. unter Zufuhr von weiterem Lösungsmittel, wie Wasser) werden dann in einem abschließenden Schritt so miteinander vermischt und entlüftet, dass eine homogene flüssige Dispersion entsteht. Dies erfolgt vorteilhaft mittels der Pulverbenetzungs- oder Dispergiermaschine, indem die besagte homogene Lösung über diese eingezogen und mit der wirkstoffhaltigen Dispersion vermischt wird und die Vermischung und Entlüftung zugleich mit einem Leitstrahlmischer (z.B. der Firma Ystral) unterstützt wird. Ein Beispiel für die zu verwendende Apparatur zeigt Fig.1 (siehe unten unter Beispiele, wo bevorzugte Bestandeile näher beschrieben sind, die auch im hier allgemein beschriebenen Verfahren verwendet werden können).

Auf diese Weise wird erreicht, dass die verteilten, mikronisierten Wirkstoffpartikel ideal in der Lösung verteilt werden und so praktisch auch ideal von funktionellem Hilfsmittel und weiteren Komponenten, wie insbesondere Bindemittel, in der Lösung umgeben sind. Dieser Zustand lässt sich durch ein Vermischen der reinen Feststoffe nicht erreichen, insbesondere auch deshalb, weil aus dem mikronisierten Wirkstoff der sehr hohe Luftanteil (in der Schüttung sind um die 90 % möglich) erst entfernt werden muss, bevor der Kontakt mit dem funktionellen Hilfsmittel und den übrigen Komponenten erfolgt, da es ansonsten zu einer starken, unerwünschten Schaumbildung kommen würde.

Vorzugsweise wird zur Herstellung der Mikropellets wie folgt vorgegangen: Der in Wasser schwer- oder unlösliche mikronisierte Wirkstoff, oder ein Gemisch von zwei oder mehr derartigen Wirkstoffen, wird in Wasser suspendiert und anschließend homogenisiert, so dass Wasser und Wirkstoff in einer praktisch idealen, homogen verteilten Dispersion vorliegen, wobei dies vorzugsweise wie oben als "erster separater Schritt" beschrieben erfolgt, insbesondere unter Vorlegen des Feststoffs in einem Pulvertrichter, Einsaugen des Feststoffs in das vorgelegte Lösungsmittel, z.B. Wasser, beispielsweise mittels eines CONTI TDS-2 der Firma Ystral bei einer Drehzahl von 4000 bis 6000 U/min, bis das gesamte Pulver eingesaugt ist, und Homogenisieren unter gleichzeitigem Entlüften, beispielsweise mit der CONTI TDS 2 und einem Leitstrahlmischer für eine vorab ermittelte und festgelegte Zeit, z.B. 1 bis 60 min, wie 10 ± 2 min. Die erhältliche Dispersion wird mit einer (insbesondere wie oben unter dem "separaten weiteren Schritt" hergestellten) Lösung der löslichen (insbesondere wasserlöslichen) funktionellen Hilfsmittel und übrigen Komponenten und/oder zusätzlichem Wasser (letzteres beispielsweise vorher, anschließend oder parallel zur genannten Lösung) zur Bildung eines Mikropellets, wie jeweils insbesondere vor- und nachstehend näher definiert, gemischt (vorzugsweise liegen Wirkstoff funktionelles Hilfsmittel und ggf. Bindemittel in den oben als bevorzugt angegebenen Mengen vor), insbesondere wie oben beschrieben, in einer möglichen bevorzugten Ausführungsform beispielsweise mittels der oben bereits genannten CONTI TDS-2. Die dabei entstehende Dispersion (Feststoffkonzentration in einem Bereich, dass die Dispersion noch gepumpt oder aber auch zerstäubt werden kann, beispielsweise von 5 bis 40 Gew.-%, beispielsweise insbesondere 15 bis 25 Gew.-%) wird (vorzugsweise nach erneutem Entlüften in Anschluss an ihre Zugabe) in einem Wirbelschichttrockner (beispielsweise in einem Verfahren und unter Verwendung einer Anlage nach EP 0 163 836, deren Inhalt hier bezüglich der verwendeten Verfahren und Apparaturen durch Bezugnahme aufgenommen wird), vorzugsweise unter Verwendung einer 2-Stoffdüse, versprüht und zu Mikropellets verarbeitet. Das Lösungsmittel (Wasser) wird während des Trocknungsprozesses durch Verdunstung entfernt.

Beispielsweise liegt das Verhältnis von Wirkstoff zu Lösungsvermittler zwischen 20:1 und 1:1 liegen, beispielsweise für eine mögliche bevorzugte Ausführungsform zwischen 10:1 und 3:1, z.B. bei ungefähr 4:1. Dies ermöglicht, Mikropellets mit hohem relativem Wirkstoffgehalt herzustellen.

Es entstehen (ohne Zugabe kernbildender Substanzen als Keime) Mikropellets mit homogener Verteilung, vergleichbar einer "festen Dispersion", d.h., die Wirkstoffanteile liegen nicht molekular verteilt, sondern in Verteilung auf der Basis der mikronisierten Teilchen vor. Die Verteilung von Wirkstoff und funktionellen Hilfsmitteln zur Formung der festen Dispersion und gegebenenfalls einem oder mehreren Bindemitteln entspricht der einer homogenen Verteilung der flüssigen Dispersion (= Matrixsystem). Entmischungsphänomene können so wirksam vermieden werden.

Sobald eine derartige "feste Dispersion" erneut mit einem Lösungsmittel (Wasser, Flüssigkeiten des Gastrointestinaltraktlumens) in Berührung gebracht wird, zerfällt das Mikropellet sofort in die einzelnen mikronisierten Feststoffteilchen des mikronisierten Wirkstoffs. Jedes einzelne mikronisierte feste Wirkstoffteilchen ist homogen von einem funktionellen Hilfsmittel zur Formung der festen Dispersion umgeben und kann so rasch in Dispersion, ja sogar in Lösung gehen. Der Wirkstoff wird so optimal resorbierbar und damit die Bioverfügbarkeit entscheidend und deutlich erhöht.

Die Eigenschaften, insbesondere das Wirkstoff-Freisetzungsprofil, des unbeschichteten Mikropelletkerns können im Rahmen des Wirbelschichtverfahrens ohne große experimentelle Schwierigkeiten durch Wahl der Komponenten und Parameter eingestellt werden, beispielsweise durch Variation der Komponenten der Zusammensetzung des Kerns und die Einstellung der Größe der Mikropellets, wobei die für das jeweils gewünschte Ziel geeigneten Verhältnisse für den Fachmann leicht zu ermitteln sind.

Der Terminus "Freisetzungsprofil" bezieht sich auf das Muster der Freisetzung des jeweiligen Wirkstoffes, beispielsweise im Darm, über die Zeit. Dies, als Maß für die Bioverfügbarkeit, kann entweder in vivo ermittelt werden, beispielsweise durch Bestimmung des Blutspiegels des Wirkstoffes, oder vorzugsweise ex vivo, beispielsweise mittels der "USP paddle"-Methode, die es ermöglicht, die Auflösungsrate des Wirkstoffes zu ermitteln.

Durch geeignete Parametervariierung (Zusammensetzung, Wirbelschichtverfahren) können jeweils besonders für die Weiterverarbeitung (z.B. Sieben, Mischen, Dosierung und Beschichtung) geeignete Mikropellets hergestellt werden. Im Gegensatz zu Extrusionsverfahren werden kugelförmige Partikel geringer Größe erzielt mit konzentrischem, homogenem Matrixaufbau. Auch mit einem Eintopfmischer mit anschließender Trocknung oder auch übliche Wirbelschichtgranulation werden ähnliche Ergebnisse nicht erreicht.

Beispielsweise wird ein USP paddle-Apparat bei 37 °C und 30 bis 100 rpm (Umdrehungen/min), beispielsweise bei 75 rpm, verwendet und die erfindungsgemäßen (unbeschichteten oder beschichteten) Mikropellets werden in (beispielsweise 900 ml) künstlicher Gastrointestinalflüssigkeit, z.B. Phosphatpuffer bei pH 6.8, künstlichem Magensaft, wie 0,1 N HCl, oder Wasser untersucht. Nach vorbestimmten Zeitpunkten (z.B. 1, 2, 5, 10, 15, 20, 25, 30 und 60 Minuten) werden Proben abgezogen und die Menge an freigesetztem Wirkstoff wird durch Bestimmung mittels Standardmethoden, wie HPLC oder Spektrophotometrie, ermittelt.

Beispielsweise zerfallen in einer möglichen bevorzugten Ausführungsform der Erfindung die Partikeln bei 37 °C unter den genannten Bedingungen so, dass nach 15 min 75 % oder mehr des Wirkstoffes (in gelöster und/oder mikronisierter Form) freigesetzt werden, nach 30 min 85 % oder mehr und nach 45 min 95 % oder mehr.

Vorzugsweise haben die erfindungsgemäß herstellbaren Mikropellets Teilchendurchmesser unterhalb von 600 µm, beispielsweise zwischen 10 und 500 µm, beispielsweise zwischen 200 und 400 µm.

Die Mikropellets gemäß der Erfindung können direkt oder nach Beschichtung zu pharmazeutischen Präparaten verarbeitet werden.

So können die Mikropellets direkt in Trockenkapseln abgefüllt werden, oder sie können gemeinsam mit anderen Hilfsstoffen zu Tabletten, insbesondere Dragées, verarbeitet werden.
Für Trockenkapseln finden beispielsweise Hartkapsein aus Gelatine Verwendung, oder auch weiche, versiegelte Kapseln aus Gelatine und einem Weichmacher, wie Glyzerin oder Sorbit. Den Mikropellets können in den Kapseln weitere Hilfsstoffe, beispielsweise Füllstoffe, wie Maisstärke, Bindemittel oder Gleitmittel, wie Talkum oder Magnesiumstearat, und gewünschtenfalls Stabilisatoren, wie Konservierungsmittel, zugesetzt werden.

Als Hilfsstoffe für Tabletten finden übliche Hilfsstoffe Verwendung, beispielsweise Trägermaterialien, wie Füllstoffe, z.B. Zucker, wie Laktose, Saccharose, Mannit oder Sorbit, Cellulosepräparate und/oder Kalziumphosphate, wie Trikalziumphosphat oder Kalziumhydrogenphosphat, und auch Bindemittel, wie Stärken, z.B. Mais-, Weizen-, Reis- oder Kartoffelstärke, Methylcellulose, Hydroxymethylcellulose, Natrium-Carboxymethylcellulose und/oder Polyvinylpyrrolidon; und gewünschtenfalls Sprengmittel, wie die oben erwähnten Stärken, auch Carboxymethylstärke, quervernetztes Polyvinylpyrrolidon, Alginsäure oder ein Salz davon, z.B. Natriumalginat. Weitere Hilfsstoffe sind insbesondere Fließreguliermittel und Gleitmittel, z.B. Kieselsäure, Talkum, Stearinsäure oder Salze davon, wie Magnesium- oder Kalziumstearat, und/oder Polyethylenglykol oder Derivate davon.

Die Tablettenkerne können mit geeigneten, gewünschtenfalls magensaftresistenten, Beschichtungen versehen werden, beispielsweise unter Verwendung von konzentrierten Zuckerlösungen, die Gummi Arabicum, Talkum, Polyvinylpyrrolidon, Polyethylenglykol und/oder Titandioxid umfassen können, oder Lacke in geeigneten organischen Lösungsmitteln oder Lösungsmittelgemischen, oder, für die Herstellung magensaftresistenter Beschichtungen, Lösungen geeigneter Cellulosepräparate, wie Acetylcellulosephthalat oder Hydroxypropylmethylcellulosephthalat. Farbstoffe oder Pigmente können den Tabletten oder Tablettenbeschichtungen zugesetzt werden, zum Beispiel für Identifikationszwecke oder um unterschiedliche Dosierungen der Wirksubstanz anzuzeigen.

Die beschriebenen Mikropellets eignen sich andererseits, wie erwähnt, besonders für die Beschichtung mit geschmacksabdeckenden und/oder magensaftresistenten Beschichtungen. Die Beschichtung ist vorzugsweise ein- oder mehr- (wie zwei-)fach.

Vorzugsweise findet sich direkt auf dem Kern des Mikropellets (mit dem Wirkstoff) eine Schutzbeschichtung, um eine Trennung des wirkstoffhaltigen Mikropelletkerns von einer weiteren äußeren magensaftresistenten geschmacksmaskierenden äußeren Beschichtung (Tastemasking Coating) sicher zu stellen. Denn bei direktem magensaftresistenten Beschichten kann es zu einem partiellen "Anlösen" des Wirkstoffs und dadurch bedingt teilweiser Diffusion des Wirkstoffs an die Oberfläche des beschichteten Mikropellets kommen, wodurch bei sehr bitter schmeckenden Wirkstoffen keine sichere Geschmacksabdichtung erzielt werden kann.

Als Schutzbeschichtung auf dem Kern kann beispielsweise eine Beschichtung mit einem (aus wässriger oder organischer Lösung aufgetragenen) Filmbildner, z.B. Cellulosederivate, wie Hydroxyethylcellulose, Hydroxypropylmethylcellulose, Celluloseacetatdibutyl- oder Celluloseacetatdicyclohexyl-aminohydroxypropylether oder Celluloseacetatphthalat, Acrylat- oder Methacrylatpolymere, Mischpolymere aus Alkyl-, wie Buytylmethacrylat und Dimethylaminomethacrylat, Schellack, Polyvinylpyrrolidon, Prolamin, Polyvinylacetat, Methacrylsäuremorpholino-N-β-ethylacrylat- oder Acrylsäuremorpholino-N-β-ethylmethacrylat-styrolacrylat-Copolymer, Mischpolymerisate aus 2-Hydroxyethyl-, 2-Hydroxypropyl-, 2-Hydroxybutyl- oder 4-Hydroxybutylester der Acryl oder Methacrylsäure, Poly(vinylacetal)dialkylaminoacetat, oder Mischungen von Rohrzucker und Montmorrillionit; oder Gemischen davon, vorgesehen sein. Dazu können Füllstoffe, wie Titandioxid, Silikate, Talkum, Kreide, Harnstoffderivate, Stärke, Alginate, Getreidemehle oder dergleichen vorliegen und gewünschtenfalls ein Weichmacher, beispielsweise Polyethylenglykol, wie PEG 6000.

Vorzugsweise liegt in der Schutzbeschichtung das Beschichtungsmittel oder Beschichtungsmittelgemisch in einem Anteil (bezogen auf die Gesamtmenge der Schutzbeschichtung) von 30 bis 90 Gew.-%, ein Füllstoff in einem Anteil von 0 bis 40, vorzugsweise von 10 bis 30 Gew.-%, vor, der Weichmacher in einem Anteil von 0 bis 30, vorzugsweise 5 bis 12 Gew.-%.

Als äußere Beschichtung (die auch alleine vorliegen kann, d.h. ohne die oben genannte innere Beschichtung) wird beispielsweise eine lipophile, insbesondere magensaftresistente Schicht gewählt, die eine ausreichende Geschmacksabdeckung und gleichzeitig ein sehr schnelles Freisetzen des Wirkstoffs bei höheren pH Werten, insbesondere von pH 6,8 oder höher, ermöglicht, deren Zusammensetzung insbesondere wie charakterisiert ist durch eine Kombination eines lipophilen Trennmittels mit einer oberflächenaktiven Substanz als Lösungsvermittler in Gegenwart einer filmbildenden Komponente.

Als filmbildende Komponente in der äußeren Beschichtung findet insbesondere eines der oben als Filmbildner erwähnten Beschichtungsmittel Verwendung, soweit es magensaftresistent ist, oder vorzugsweise ein Alkylacrylat-Polymeres, wie Eudragit L 30 D-55® (Röhm) (Copolymerisat aus Methacrylsäure und Ethacrylat im Verhältnis 1:1).

Als lipophiles Trennmittel kann beispielsweise ein Ester, zum Beispiel ein Tri-C₁-C₇ Alkylcitrat, wie Triethylcitrat, beispielsweise im Gewichtsverhältnis von 1:50 bis 5:1, oder auch andere, homogene wässrige Emulsionen bildende Substanzen, oder Gemische von zwei oder mehr davon, eingesetzt werden.

Das lipophile Trennmittel in der äußeren Beschichtung liegt vorzugsweise, bezogen auf die Gewichtsanteile der Komponenten der äußeren Schicht, in einem Anteil von 0,05 bis 50 Gew.-% vor, die filmbildende Komponente in einem Anteil von 40 bis 99,05, wobei diese Komponenten zusammen 100 % ergeben.

Das Beschichten (Coating) als nachfolgender Schritt nach der Herstellung der Mikropellets, wie oben beschrieben, findet vorzugsweise ebenfalls im Wirbelstromverfahren statt (nach dem Wurster-Verfahren, beispielsweise in einer möglichen bevorzugten Ausführungsform der Erfindung in einer Wirbelschichtanlage wie in US 5,236,503 und US 5,437,889, die hier diesbezüglich durch Bezugnahme aufgenommen werden, beschrieben): hierbei wird mit Sprühdüsen im Boden die Beschichtungsflüssigkeit, in der die Beschichtungsmittel gelöst oder emulgiert sind, im Gleichstrom mit den zu beschichtenden Mikropellets versprüht. Besonders vorteilhaft ist es, wenn die Düse so ausgeführt wird, dass ein Anlagern von kleinen Partikeln an der Düsenspitze verhindert wird. Dies wird beispielsweise erreicht, indem die Sprühdüse von einem zylindrischen, unten offenen Rohr umgeben wird, welches die Wirkung hat, dass die Prozessluft, die in diesem Rohr auch noch beschleunigt wird, um die Düsenspitzen ein Luftpolster ausbildet, wodurch insbesondere kleine Partikel daran gehindert werden, sich an die Düsenspitze anzulagern.

Hier wird beispielsweise erst eine (innere) Schutzbeschichtung mit den oben als bevorzugt bezeichneten Komponenten aufgetragen, anschließend (im selben Ansatz oder nach Zwischenisolierung des Produktes = einfach beschichtete Mikropellets) eine oder mehrere weitere Beschichtungen, vorzugsweise eine weitere äußere Beschichtung, vorzugsweise wie oben beschrieben. Alternativ wird nur eine der oben als äußere Beschichtungen bezeichneten Beschichtungen ein- oder mehrfach aufgetragen.

Durch die genannten Beschichtungsverfahren ist ein gleichmäßiges Beschichten der Mikropellets möglich. Eine oder auch zwei oder mehrere Schichten werden als sehr dünner Film gleichmäßig und vollständig die Pelletoberfläche abdeckend aufgebracht. Da, wie oben beschrieben, die erfindungsgemäß hergestellten Mikropellets für die Beschichtung sehr günstige Eigenschaften haben, kann die Menge an Beschichtungsmaterial minimiert werden, was insbesondere für das Beschichten von kleinen Partikeln/Mikropellets sehr vorteilhaft ist. Daraus resultieren folgende Produktvorteile:
- geringer Verbrauch von Beschichtungsmaterial
- kurze Prozesszeiten
- geringe Filmdicken ergeben letztendlich auch kleine Pelletgrößen für die beschichteten Mikropellets. Dies ist von Bedeutung in Verbindung mit gewünschten kleinen Parikelgrößen, wie dies z.B. bei Trinksuspensionen erforderlich ist.
- Gezieltes und einfaches Aufbringen von Mehrschicht-Beschichtungen (z.B. zweifache Beschichtung).

Bevorzugt ist ein Verfahren, das sowohl die oben beschriebene Herstellung des Mikropellets als auch dessen Beschichtung, insbesondere mit zwei Beschichtungen, einer Schutzschicht und einer äußeren Schicht, beinhaltet.

Besonders bevorzugt sind auch die nach diesem Verfahren erhältlichen beschichteten Mikropellets. Eine weitere bevorzugte Ausführungsform der Erfindung bezieht sich auf pharmazeutische Formulierungen, welche die wie beschrieben hergestellten erfindungsgemäßen unbeschichteten oder insbesondere beschichteten Mikropellets beinhalten. Hier stehen insbesondere enteral, vor allem oral, verabreichbare pharmazeutische Formulierungen im Vordergrund, sei es in Form von Trinksuspensionen oder per Schlauch direkt in den Magen- oder Darmtrakt einführbaren Suspensionen, oder (für die rektale Anwendung) Suspensionen für Klistiere oder dergleichen, oder in Form von Suspensionen für oral verwendbare Kapseln, oder auch für Tabletten, sowie jeweils für die Herstellung derartiger pharmazeutischer Formulierungen. Diese Formulierungen werden nach üblichen Verfahren hergestellt.

Durch die Wahl der beteiligten Komponenten kann eine pH-abhängig möglichst rasche Freisetzung bei höheren pH-Werten (wie sie beispielsweise im Darm vorliegen), beispielsweise pH-Werte von 6,8 oder mehr, erreicht werden, während bei geringeren pH-Werten, beispielsweise pH 5,5 oder niedriger, keine Freisetzung erfolgt.

Die Erfindung kann in einer weiteren Ausführungsform auch eine Apparatur wie in Fig. 1 gezeigt und/oder der obigen Beschreibung von Fig. 1 allgemein beschrieben sowie deren Verwendung zum Dispergieren mikronisierter Wirkstoffe, wie oben und unten beschrieben, insbesondere im Rahmen der erfindungsgemäßen Mikropelletherstellung, betreffen.

Die vorstehenden Definitionen bestimmter Begriffe können auch einzeln oder zu mehreren zur genaueren Definition von allgemeineren Begriffen in den Ansprüchen oder anderen Ausführungsformen der Erfindung verwendet werden, was zu besonders bevorzugten Ausführungsformen der Erfindung führt.

Ganz besonders bevorzugt sind die in den Beispielen genannten Ausführungsformen der Erfindung.

Die nachfolgenden Beispiele dienen der Illustration der Erfindung und schränken ihren Umfang nicht ein (alle %-Angaben sind in Gew.-%):

### Beispiel 1: Mikropellets mit Makrolidantibiotikum (z.B. Azithromycin oder insbesondere Clarithromycin):

Nach dem Wirbelschichtverfahren werden folgendermaßen Mikropellets hergestellt:

### 1. Herstellung einer flüssigen Dispersion enthaltend schwer wasserlöslichen Wirkstoff

Bezugnahmen erfolgen auf Fig. 1.

Mikronisiertes Makrolidantibiotikum (z.B. Azithromycin oder insbesondere Clarithromycin) (Korngröße < 30 µm) wird mittels eines CONTI TDS-2 (= Pulverbenetzungsmaschine der Fa. Ystral) **4** (siehe Fig 1) pulverförmig aus einem Pulvertrichter in vorgelegtes Wasser (2-fache Wassermenge, d.h. Menge des Wirkstoffs 12 kg, Menge Wasser 24 kg) eingezogen, wobei darauf geachtet wird, dass keine Luft mit eingezogen wird, und anschließend zusammen mit einem Leitstrahlmischer (Jetstreammixer der Firma Ystral) 2 vermischt, homogenisiert (Dauer 10 min) und entlüftet.

Dies geschieht insbesondere mit der in Fig. 1 gezeigten Anlage. Zur Inbetriebnahme ist das Ventil **9** geöffnet, alle anderen Ventile sind geschlossen. Ein Kühlmittelversorgungssystem für eine Gleitringdichtung (beide nicht gezeigt) wird eingeschaltet. Registriert ein Druckwächter (nicht gezeigt) einen ausreichend großen Druck in der Kühlmittelversorgungsleitung, wird die Pumpe freigegeben. Ventil **8** wird geöffnet und die Pumpendrehzahl zwischen 4000 und 6000 U/min eingestellt. Gleichzeitig wird der Leitstrahlmischer mit 1500 bis 5000 U/min eingeschaltet. Zum Einziehen des Wirkstoffes wird das Ventil **5** geöffnet, bis der Wirkstoff aus dem Pulvertrichter **1** eingezogen ist. Während des Pulvereinzugs wird am Pulvertrichter ein Intervallklopfer (nicht gezeigt) in Betrieb gesetzt. Zum Homogenisieren wird das Ventil **5** geschlossen und die CONTI TDS-2 mit einer Drehzahl zwischen 4000 und 6000 eingestellt. Die Temperatur der Suspension wird überwacht, um einen bestimmten Wert abhängig von der Viskosität (z.B. 60°C) nicht zu überschreiten. Das als Kugelventil ausgeführte Ventil 5 ist vorteilhaft als Kugelventil und sehr schnell öffen- und schließbar (um den Einziehvorgang beispielsweise im Falle einer Kanalbildung im Trichter mit Gefahr des Lufteinzugs rasch unterbrechen zu können) ausgeführt und wird zum Einziehen in die Anlage geöffnet.

In einem zweiten Ansatzbehälter wird eine wässrige Tensidlösung mit zusätzlichem Bindemittel angesetzt und in Lösung gebracht: Vorgelegtes Wasser (61,225 kg) wird mit dem Tensid (3 kg Poloxamer 188 = Pluronic®) und 2,045 Bindemittel (Polyvinylpyrrolidon) vermischt.

Die klare Lösung wird mittels der CONTI TDS-2 der Wirkstoffdispersion zugeführt und unter Einsatz des Leitstrahlmischers vermischt, homogenisiert und entlüftet. Dabei wird die CONTI TDS-2 mit einer Drehzahl zwischen 2000 und 6000 U/min betrieben. Das Ventil 6 wird geöffnet, bis die gewünschte Menge an Tensidlösung mit Bindemittel eingezogen ist. Dann wird das Ventil geschlossen. Der Leitstrahlmischer arbeitet hier bei einer Drehzahl von 300 bis 1500 U/min.

Es folgt ein wiederholtes Durchlaufen der folgenden Misch- und Entlüftungssequenz: Das Mischen wird zunächst mit der CONTI TDS-2 bei 4000 bis 6000 U/min durchgeführt. Der anschließend wirksame Leitstrahlmischer arbeitet bei einer Drehzahl von 3000 bis 5000 U/min.

Unter Öffnung der Ventile **8, 9** und **10** wird das Produkt in den Ansatzbehälter **3** abgepumpt. Zur Restentleerung der CONTI TDS-2 wird das Ventil **11** geöffnet und ein geeignetes Auffanggefäß unter den Auslass gehalten. Die CONTI TDS-2 und der Leitstrahlmischer sind ausgeschaltet.

Anschließend kann die so erhaltene flüssige Dispersion direkt versprüht werden zur Pelletherstellung.

### 2. Pelletherstellung

Die Pelletherstellung erfolgt durch Sprühgranulation, indem die flüssige Wirkstoffdispersion von unten in eine leere Wirbelschichtanlage eingesprüht wird. Sobald die Partikel die erwünschte Partikelgröße erreicht haben, werden diese mittels eines Zick-Zack-Sichters aus der Anlage entnommen (vgl EP 0 163 836, EP 0 332 031). Als Anlage wird bvorzugsweise eine GPCG 30 mit WSA-Modul (Wirbelschicht-Sprühagglomeration) (beide Fa. Glatt, Binzen, Deutschland) eingesetzt (GPCG = Glatt Partikel Coater Granulator).

| | |
|---|---|
| Zulufttemperatur: | 120°C |
| Zuluftvolumen: | 550m³/h |
| Produkttemperatur: | 72 °C |

Die Zielgröße der Pellets liegt zwischen 200 und 400 µm.

Zusammensetzung der unbeschichteten Pellets (unter Annahme von 100 % Makrolidantibiotikum in der Ausgangscharge): Makrolidantibiotikum 70 %, Pluronic® 18 %, Polyvinylpyrrolidon K30 12 %.

### 3. Coating (Taste Masking)

Das Aufbringen einer Coating-Schicht erfolgt in einem GPCG 30 mit 18 "HS Wurster (; HS = High Speed Wurster, vgl. US 5,236,503 und/oder US 5,437,889; Fa. Glatt, Binzen, Deutschland)

| | |
|---|---|
| Vorgelegte Menge an Pellets: | 25 kg |
| Aufgetragene Coatingmenge: | 12,5 kg (entspricht einem Gewichtzuwachs von 50 % bezogen auf die Pellets) |

### Zusammensetzung des Coating-Materials:

| | |
|---|---|
| Eudragit® L 30 D 55 (Fa. Degussa) | 83,89 % |
| Triethylcitrat (Fa. Morflex) | 12,58 % |
| Glycerolmonostearat (Fa. Cognis) | 2,52 % |
| Tween 80 (Fa. Uniquema) | 1,01 % |

### Prozessparameter:

| | |
|---|---|
| Zulufttemperatur | 70 °C |
| Zuluftmenge | 1000 m³/h |
| Produkttemperatur | 42 °C |

Ergebnisse der In-vitro-Freisetzung: Nach der oben beschriebenen US-paddle-Methode werden bei 37 °C und 75 rpm nach 15 min mehr als 75 % des Wirkstoffes freigesetzt. Die resultierenden beschichteten Pellets zeigen nur leichte Bitterkeit und somit eine tolerierbare Geschmacksabschirmung.

### Beispiel 2: Zwei-Schicht-Beschichtung:

1. Beschichtung: Polyvinyl-pyrrolidon-organisch
2. Beschichtung: Eudragit L30 D-55 + 10 % Triethylcitrat

Nach dem in Beispiel 1 hergestellten Verfahren gewonnene Mikropellets werden nach dem Wurster-Verfahren der Reihe nach mit der 1. (inneren), dann der 2. (äußeren) Beschichtung versehen.

Ergebnis: gegenüber Beispiel 1 verbesserte Geschmacksabdeckung; Freisetzungsprofil im Rahmen des US paddle-Tests bei 75 rpm und pH 6,8: mehr als 75 % nach 15 min.

## Patentansprüche

1. Verfahren zur Herstellung von einen oder mehrere schwerlösliche Wirkstoffe enthaltenden Mikropellets, welche aus folgenden Komponenten besteht:
(i) den pharmakologischen Wirkstoff in mikronisierter Form in einem Anteil von 10 bis 99 Gew.-%;
(ii) funktionelle Hilfsmittel zur Formung der festen Dispersion in einem Anteil von 1 bis 90 Gew.-%, mit oder ohne
(iii) ein Bindemittel in einem Anteil von 0 bis 20 Gew.-%,
bei dem aus Dispersionen mikronisierter Teilchen in Gegenwart des funktionellen Hilfsmittels zur Formung einer festen Dispersion derartiger Teilchen, wobei diese funktionellen Hilfsmittel und die übrigen genannten Komponenten zur Bildung der Mikropellets in gelöster oder ebenfalls dispergierter Form vorliegen, durch Sprühgranulation im Wirbelschichtverfahren die Mikropellets hergestellt werden.

2. Verfahren nach Anspruch 1, wobei das Gewichtsverhältnis von funktionellem Hilfsmittel zur Formung der festen Dispersion und Wirkstoff zwischen 20:1 und 1:100, beispielsweise zwischen 5:1 und 1:10 liegt.

3. Verfahren nach einem der Ansprüche 1 oder 2, wobei der Wirkstoff in mikronisierter Form mit einer Korngröße von 30 µm oder weniger, insbesondere zwischen 0,1 und 30 µm, vorliegt.

4. Verfahren nach einem der Ansprüche 1 bis 3, wobei als funktionelles Hilfsmittel zur Formung der festen Dispersion ein oder mehrere Lösungsvermittler vorliegen, insbesondere ein oder mehrere Polyoxypropylenpolyoxyethylenkondensate oder - blockpolymerisate, Fettsäurepolyglykolether, Alkylphenol-polyethylenglykolether, Triglyceride, anionische Tenside, kationische Tenside, amphotere Detergentien oder nichtionische Tenside, oder vorzugsweise ein Polyoxypropylen-oxyethylen(block)-polymerisat.

5. Verfahren nach einem der Ansprüche 1 bis 4, worin als schwerlöslicher Wirkstoff ein oder mehrere aus Makrolidantibiotika, insbesondere Azithromycin, schwer wasserlöslichen antiviralen Therapeutika, schwer wasserlöslichen Analgetika, schwer wasserlöslichen Herz-Kreislaufmitteln, schwer wasserlöslichen Antiphlogistika und schwer wasserlöslichen Krebstherapeutika ausgewählte Wirkstoffe vorliegen.

6. Verfahren nach einem der Ansprüche 1 bis 5, worin als schwerlöslicher Wirkstoff Clarithromycin vorliegt.

7. Verfahren nach einem der Ansprüche 1 bis 5 oder 6, **dadurch gekennzeichnet, dass** die zu pelletierenden Feststoffe als flüssige Dispersion, die den mikronisierten Wirkstoff und gegebenenfalls funktionelle Hilfsmittel zur Formung einer festen Dispersion und gewünschtenfalls ein Bindemittel enthält, von unten in eine zu Beginn des Prozesses leere Wirbelschichtanlage eingesprüht wird;
durch die Sprühgranulation der Dispersion Startkeime für die Pelletierung ohne Vorlage von anderem inertem Material gebildet werden;
und die hergestellten Mikropellets während des Prozesses über eine Klassiereinrichtung, insbesondere einen Windsichter, in erster Linie einen Zick-Zack-Sichter, gesichtet und bei Erreichen einer vordefinierten Pelletgröße aus dem Sichter entnommen werden.

8. Verfahren zur Herstellung einer Dispersion eines mikronisierten Wirkstoffs, wobei
in einem ersten separaten Schritt eine homogene Suspension des mikronisierten Wirkstoffs in Wasser hergestellt wird, indem der mikronisierte schwerlösliche, insbesondere nicht wasserlösliche Wirkstoff, mehrere entsprechende Wirkstoffe oder ein entsprechendes Wirkstoffgemisch mittels einer Pulverbenetzungs- oder Dispergiermaschine, und einem Mischer zum Homogenisieren bzw. Entlüften der Dispersion in Wasser unter Entlüftung und Homogenisierung suspendiert wird;
in einem separaten weiteren Schritt eine Lösung der löslichen, insbesondere wasserlöslichen, funktionellen Hilfsmittel und übrigen Komponenten zur Bildung eines Mikropellets, wie in einem der Ansprüche 1 bis 6 definiert, in einem Lösungsmittel hergestellt wird, bis die Lösung klar wird;
und die Dispersion aus dem ersten Schritt und die homogene Lösung aus dem weiteren Schritt in einem abschließenden Schritt so miteinander vermischt und entlüftet werden, dass eine homogene flüssige Dispersion entsteht, vorteilhaft mittels der Pulverbenetzungs- oder Dispergiermaschine, indem die besagte homogene Lösung über diese eingezogen und mit der wirkstoffhaltigen Dispersion vermischt wird und die Vermischung und Entlüftung zugleich mit einem Leitstrahlmischer unterstützt wird.

9. Verfahren nach einem der Ansprüche 1 bis 5, 7 oder 6, **dadurch gekennzeichnet, dass** eine nach Anspruch 8 erhältliche Dispersion in einem Wirbelschichttrockner versprüht wird, wobei das Lösungsmittel während des Trocknungsprozesses durch Verdunstung entfernt wird.

10. Verfahren nach einem der Ansprüche 1 bis 5, 7, 6 oder 9, wobei die Mikropellets folgende Komponenten enthalten:
(i) den pharmakologischen Wirkstoff in mikronisierter Form in einem Anteil von 20 bis 90 Gew.-%;
(ii) funktionelle Hilfsmittel zur Formung der festen Dispersion in einem Anteil von 1 bis 50 Gew.-%, und
(iii) gewünschtenfalls ein Bindemittel in einem Anteil von 5 bis 15 Gew.-%.

11. Mikropellet, erhältlich nach einem Verfahren gemäß einem der Ansprüche 1 bis 5, 7, 6 oder 9.

12. Mikropellet, erhältlich nach dem Verfahren gemäß Anspruch 10.

13. Mikropellet nach einem der Ansprüche 11 oder 12 mit einem Durchmesser von 0,1 bis 500 µm, vorzugsweise in Kugelform.

14. Mikropellets nach einem der Ansprüche 11 bis 13, worin maximal 25 Gew.-% der Pellets einen Durchmesser aufweisen, der um mehr als 25 % (±) vom mittleren Durchmesser aller Pellets abweicht.

15. Pharmazeutische Formulierung, beinhaltend Mikropellets nach einem der Ansprüche 11 bis 14.

16. Verfahren zur Herstellung von beschichteten Mikropellets, beinhaltend die Herstellung eines Mikropellets nach einem der Ansprüche 1 bis 5, 6, 7 oder 9, **dadurch gekennzeichnet, dass** nach dem Herstellen der Pellets gemäß einem der Ansprüche 1 bis 5, 6, 7 oder 9 in einem nachfolgenden Schritt das Beschichten vorzugsweise ebenfalls im Wirbelstromverfahren durchgeführt wird, wobei mit Sprühdüsen im Boden die Beschichtungsflüssigkeit, in der die Beschichtungsmittel gelöst oder emulgiert sind, im Gleichstrom mit den zu beschichtenden Mikropellets versprüht werden.

17. Verfahren nach Anspruch 16, wobei erst eine innere Schutzbeschichtung, anschließend, im selben Ansatz oder nach Zwischenisolierung des Zwischenproduktes, d.h. einfach beschichteter Mikropellets, eine oder mehrere weitere Beschichtungen aufgetragen werden.

18. Beschichtete Mikropellets, erhältlich nach dem Verfahren nach einem der Ansprüche 16 oder 17.

19. Beschichtete Mikropellets nach Anspruch 18, welche zwei Beschichtungen aufweisen, insbesondere eine innere Schutzbeschichtung und eine äußere magensaftresistente Beschichtung.

20. Beschichtete Mikropellets nach einem der Ansprüche 18 oder 19, welche beim US paddle-Test bei 75 rpm in Lösung bei pH von 6,8 oder höher in 15 Minuten eine Wirkstofffreisetzung von 75 % oder mehr zeigen.

21. Pharmazeutische Formulierung, beinhaltend ein beschichtetes Mikropellet nach einem der Ansprüche 18 oder 19.

## Claims

1. Process for preparing micropellets containing one or more active substances that are poorly soluble, consisting of the following components:
(i) the pharmacological active substance in micronised form in an amount of from 10 to 99 wt.%;
(ii) functional adjuvants for forming the solid dispersion in an amount of from 1 to 90 wt.%, with or without
(iii) a binder in an amount of from 0 to 20 wt.%,
wherein the micropellets are produced by spray granulation by the fluidised bed method from dispersions of micronised particles in the presence of the functional adjuvant for forming a solid dispersion of such particles, these functional adjuvants and the other components mentioned above for forming the micropellets being present in dissolved or also in dispersed form.

2. Process according to claim 1, wherein the ratio by weight of functional adjuvant for forming the solid dispersion and the active substance is between 20:1 and 1:100, for example between 5:1 and 1:10.

3. Process according to one of claims 1 or 2, wherein the active substance is present in micronised form with a particle size of 30 µm or less, more particularly between 0.1 and 30 µm.

4. Process according to one of claims 1 to 3, wherein one or more solubilising agents are present as the functional adjuvant for forming the solid dispersion, particularly one or more polyoxypropylene-polyoxyethylene condensates or block polymers, fatty acid polyglycol ethers, alkylphenol-polyethyleneglycol ethers, triglycerides, anionic surfactants, cationic surfactants, amphoteric detergents or nonionic surfactants, or preferably a polyoxypropylene-oxyethylene (block) polymer,

5. Process according to one of claims 1 to 4, wherein one or more active substances selected from among macrolide antibiotics, particularly azithromycin, poorly water-soluble anti-viral therapeutic agents, poorly water-soluble analgesics, poorly water-soluble cardiovascular agents, poorly water-soluble antiinflammatories and poorly water-soluble anti-cancer drugs are present as the poorly soluble active substance.

6. Process according to one of claims 1 to 5, wherein clarithromycin is present as the poorly soluble active substance.

7. Process according to one of claims 1 to 5 or 6, **characterised in that** the solids to be pelleted are sprayed from below into a fluidised bed apparatus which is empty at the start of the process, in the form of a liquid dispersion which contains the micronised active substance and optionally functional adjuvants in order to form a solid dispersion and optionally a binder;
as a result of the spray granulation of the dispersion, starter germs are formed for the pelleting without any other inert material being supplied;
and the micropellets produced during the process are sifted through a grading device, particularly a wind sifter, primarily a zig-zag sifter, and once a redefined pellet size has been reached they are removed from the sifter.

8. Process for preparing a dispersion of a micronised active substance, wherein
in a first separate step a homogeneous suspension of the micronised active substance is produced in water, by suspending the micronised poorly soluble, more particularly water-insoluble active substance, several such active substances or a corresponding active substance mixture in water, while eliminating air and carrying out homogenisation, using a powder wetting or dispersing machine, and a mixer for homogenising or de-aerating the dispersion;
in a separate further step a solution of the soluble, particularly water-soluble, functional adjuvants and other components for forming a micropellet as defined in one of claims 1 to 6 is prepared in a solvent until the solution becomes clear;
and in a final step the dispersion from the first step and the homogeneous solution from the further step are mixed together and de-aerated such that a homogeneous liquid dispersion is formed, advantageously by means of the powder wetting or dispersing machine, by taking in the said homogeneous solution through said machine and mixing it with the active substance-containing dispersion, the mixing and de-aeration simultaneously being assisted by a jet stream mixer.

9. Process according to one of claims 1 to 5, 7 or 6, **characterised in that** a dispersion that may be obtained according to claim 8 is sprayed in a fluidised bed dryer, the solvent being eliminated during the drying process by evaporation.

10. Process according to one of claims 1 to 5, 7, 6 or 9, wherein the micropellets contain the following components:
(i) the pharmacological active substance in micronised form in an amount of from 20 to 90 wt.%;
(ii) functional adjuvants for forming the solid dispersion in an amount of from 1 to 50 wt.%, and
(iii) if desired a binder in an amount of from 5 to 15 wt.%.

11. Micropellet, obtainable by a process according to one of claims 1 to 5, 7, 6 or 9.

12. Micropellet, obtainable by the process according to claim 10.

13. Micropellet according to one of claims 11 or 12 having a diameter of 0.1 to 500 µm, preferably in spherical form.

14. Micropellets according to one of claims 11 to 13, wherein at most 25 wt.% of the pellets have a diameter which deviates from the mean diameter of all the pellets by more than 25% (±).

15. Pharmaceutical formulation containing micropellets according to one of claims 11 to 14.

16. Process for producing coated micropellets, comprising preparing a micropellet according to one of claims 1 to 5, 6, 7 or 9, **characterised in that** after the preparation of the pellets according to one of claims 1 to 5, 6, 7 or 9, in a subsequent step, coating is preferably also carried out by the fluidised bed method, while the coating liquid in which the coating agents are dissolved or emulsified is sprayed in cocurrent flow with the micropellets that are to be coated by means of spray nozzles in the base.

17. Process according to claim 16, wherein first of all an inner protective coating and then, in the same batch or after intermediate isolation of the intermediate product, i.e. micropellets with a single coating, one or more further coatings are applied.

18. Coated micropellets which may be obtained by the process according to one of claims 16 or 17.

19. Coated micropellets according to claim 18 which have two coatings, particularly an inner protective coating and an outer coating resistant to gastric juices.

20. Coated micropellets according to one of claims 18 or 19 which exhibit a release of active substance of 75% or more in 15 minutes in the US paddle test at 75 rpm in solution at pH 6.8 or higher.

21. Pharmaceutical formulation containing a coated micropellet according to one of claims 18 or 19.

## Revendications

1. Procédé de production de micropellets comprenant une ou plusieurs substances actives difficilement solubles, qui se compose des composants suivants :
(i) la substance active pharmacologique sous la forme micronisée dans une proportion de 10 à 99 % en poids ;
(ii) des moyens auxiliaires fonctionnels pour former la dispersion solide dans une proportion de 1 à 90 % en poids avec ou sans
(iii) un liant dans une proportion de 0 à 20 % en poids,
pour lequel à partir de dispersions de particules micronisées, les micropellets sont produits en présence du moyen auxiliaire fonctionnel pour former une dispersion solide de telles particules, ces moyens auxiliaires fonctionnels et les autres composants cités pour former les micropellets se présentant sous la forme dissoute ou également dispersée, par granulation vaporisée dans un procédé par lit fluidisé.

2. Procédé selon la revendication 1, dans lequel le rapport de poids entre le moyen auxiliaire fonctionnel pour former la dispersion solide et la substance active est compris entre 20:1 et 1:100, par exemple entre 5:1 et 1:10.

3. Procédé selon l'une quelconque des revendications 1 ou 2, dans lequel la substance active se présente sous la forme micronisée avec une granulation de 30 µm ou moins, en particulier comprise entre 0,1 et 30 µm.

4. Procédé selon l'une quelconque des revendications 1 à 3, dans lequel un ou plusieurs agents de solubilisation se présentent comme moyen auxiliaire fonctionnel pour former la dispersion solide, en particulier un ou plusieurs condensats ou polymères bloc de polyoxypropylène et polyoxyéthylène, un éther de polyglycol d'acide gras, un éther d'alkylphénol et de polyéthylèneglycol, des triglycérides, des agents tensioactifs anioniques, des agents tensioactifs cationiques, détergents amphotères ou des agents tensioactifs non-ioniques ou de préférence un polymère (bloc) de polyoxypropylène et d'oxyéthylène.

5. Procédé selon l'une quelconque des revendications 1 à 4, dans lequel une ou plusieurs substances actives sélectionnées parmi les antibiotiques macrolides, en particulier l'azithromycine, les médicaments antiviraux peu solubles dans l'eau, les analgésiques difficilement solubles dans l'eau, les toniques cardio-vasculaires difficilement solubles dans l'eau, les antiphlogistiques difficilement solubles dans l'eau et les médicaments contre le cancer difficilement solubles dans l'eau.

6. Procédé selon l'une quelconque des revendications 1 à 5, dans lequel de la clarithromycine se présente comme substance active difficilement soluble.

7. Procédé selon l'une quelconque des revendications 1 à 5 ou 6, **caractérisé en ce que** les substances solides à pelleter comme dispersion liquide qui contient la substance active micronisée et éventuellement des moyens auxiliaires fonctionnels pour former une dispersion solide et si souhaité un liant, sont pulvérisées par le bas dans une installation à lit fluidisé vide au début du processus ;
par la granulation par vaporisation de la dispersion, des germes de départ sont formés pour la pelletisation sans présentation d'autre matière inerte ;
et les micropellets produits sont triés pendant le processus par un dispositif de triage, en particulier un séparateur à air, en premier lieu un séparateur en zigzag et sont retirés du séparateur lors de l'atteinte d'une grandeur de pellet prédéfinie.

8. Procédé de production d'une dispersion d'une substance active micronisée, dans lequel
dans une première étape séparée, une suspension homogène de la substance active micronisée est produite dans l'eau, en ce que la substance active micronisée, difficilement soluble, en particulier non soluble dans l'eau, plusieurs substances actives correspondantes ou un mélange de substances actives correspondant est mis en suspension tout en aérant et homogénéisant au moyen d'une machine d'imprégnation de poudre ou d'un disperseur, et d'un mélangeur pour l'homogénéisation ou l'aération de la dispersion dans l'eau ;
dans une autre étape séparée, une solution de moyens auxiliaires fonctionnels, solubles, en particulier solubles dans l'eau et d'autres composants pour la formation d'un micropellet, comme définis dans l'une quelconque des revendications 1 à 6, est produite dans un solvant, jusqu'à ce que la solution devienne claire ;
et la dispersion de la première étape et la solution homogène de l'autre étape se mélangent et sont aérées dans une dernière étape de sorte qu'une dispersion liquide homogène apparaisse, avantageusement au moyen de la machine d'imprégnation de poudre ou du disperseur en ce que ladite solution homogène est alimentée par celle-ci et mélangée avec la dispersion contenant les substances actives et le mélange et l'aération sont assistés en même temps par un mélangeur à faisceau guidant.

9. Procédé selon l'une quelconque des revendications 1 à 5, 7 ou 6, **caractérisé en ce qu'**une dispersion pouvant être obtenue selon la revendication 8, est pulvérisée dans un sécheur en lit fluidisé, le solvant étant retiré pendant le processus de séchage par évaporation.

10. Procédé selon l'une quelconque des revendications 1 à 5, 7, 6 ou 9, dans lequel les micropellets contiennent les composantes suivantes :
(i) la substance active pharmacologique sous la forme micronisée dans une proportion de 20 à 90 % en poids ;
(ii) des moyens auxiliaires fonctionnels pour former la dispersion solide dans une proportion de 1 à 50 % en poids, et
(iii) si souhaité un liant dans une proportion de 5 à 15 % en poids.

11. Micropellet pouvant être obtenu selon un procédé selon l'une quelconque des revendications 1 à 5, 7, 6 ou 9.

12. Micropellet pouvant être obtenu selon le procédé selon la revendication 10.

13. Micropellet selon l'une quelconque des revendications 11 ou 12 d'un diamètre de 0,1 à 500 µm, de préférence de forme sphérique.

14. Micropellets selon l'une quelconque des revendications 11 à 13, dans lequel 25 % en poids au maximum des pellets présentent un diamètre qui diverge de plus de 25 % (±) du diamètre moyen de tous les pellets.

15. Formulation pharmaceutique comprenant des micropellets selon l'une quelconque des revendications 11 à 14.

16. Procédé de production de micropellets revêtus, comprenant la production d'un micropellet selon l'une quelconque des revendications 1 à 5, 6, 7 ou 9, **caractérisé en ce qu'**après la production des pellets selon l'une quelconque des revendications 1 à 5, 6, 7 ou 9, le revêtement est réalisé de préférence également dans le procédé utilisant les courants de Foucault dans une étape suivante, dans lequel le liquide de revêtement, dans lequel les moyens de revêtement sont dissous ou émulsionnés, est vaporisé avec des buses de pulvérisation dans le fond dans le même flux continu que les micropellets à revêtir.

17. Procédé selon la revendication 16, dans lequel en premier lieu un revêtement de protection intérieur, puis dans la même préparation ou après une isolation intermédiaire du produit intermédiaire, c'est-à-dire des micropellets revêtus simplement, un ou plusieurs autres revêtements sont appliqués.

18. Micropellets revêtus pouvant être obtenus selon le procédé selon l'une quelconque des revendications 16 ou 17.

19. Micropellets revêtus selon la revendication 18, qui présentent deux revêtements, en particulier un revêtement de protection intérieur et un revêtement extérieur résistant au milieu ou suc gastrique.

20. Micropellets revêtus selon l'une quelconque des revendications 18 ou 19 qui montrent lors d'un test d'agitation américain à 75 tr/min dans une solution à un pH de 6,8 ou supérieur pendant 15 minutes, une libération de substance active de 75 % ou plus.

21. Formulation pharmaceutique comprenant un micropellet revêtu selon l'une quelconque des revendications 18 ou 19.
